# EUROPEAN PATENT APPLICATION

(11) **EP 1 536 226 A1**
(43) Date of publication of application: **01.06.2005**
(21) Application number: 03792833.0
(22) Date of filing: 25.08.2003
(51) Int. Cl.: G01N 27/64, G01N 33/68, H01J 49/10, H01J 49/04

(54) **METHOD OF ANALYZING PROTEIN USING LASER ABLATION**

(30) Priority: 26.08.2002 JP 2002245706
(71) Applicant: Hayashizaki, Yoshihide, Tsukuba-shi, Ibaraki 305-0061 (JP)
(72) Inventor: HAYASHIZAKI, Yoshihide, Tsukuba-shi, Ibaraki 305-0061 (JP); TANIHATA, Isao, Iruma-gun, Saitama 350-0435 (JP)
(74) Representative: Grünecker, Kinkeldey, Stockmair & Schwanhäusser Anwaltssozietät
(86) International application number: PCT/JP2003/010706
(87) International publication number: WO 2004/019026

(57) **Abstract**

A method of analyzing a protein using laser ablation which comprises, in order to simultaneously atomize the atoms constituting the protein to be analyzed and ionize the same using a single laser source, irradiating the protein with laser beams and thus ablating the protein to thereby atomize the protein into the constituting elements and ionize the thus atomized constituting elements, and then analyzing the constituting elements thus ionized. The laser beams for ablating the subject protein are ultra-short pulse laser beams with which a chip having the protein fixed thereon is irradiated. Thus, the protein fixed on the chip is ablated with the ultra-short pulse laser beams so that the protein is atomized into the constituting elements and, at the same time, ionized followed by the analysis of the constituting elements thus ionized.

## Description

### Technical Field

The present invention relates to a method of analyzing protein using laser ablation, more particularly to a method of analyzing protein using laser ablation, which is capable of significantly improving analysis efficiency comparing to conventional ones. For example, the invention relates to a method of analyzing protein using laser ablation, which is preferable for use in mass spectrometry of protein contained in a specimen taken from a living body.

### Background Art

In recent years, the application range of mass spectrometry has rapidly spread from the field of physics and chemistry to the field of life science such as medical science and biochemistry. Particularly, its development is remarkable in the decision analysis of molecular weight of protein and the decision analysis of amino-acid sequence.

The principle of such mass spectrometry is that a sample is ionized in various methods, ions obtained by ionization are separated by mass/charge, and the intensity of each separated ion is measured.

Meanwhile, a conventional mass spectrometry of protein has been one that electrons were added to protein itself to be ionized and its mass was analyzed, or a molecule having high molecular weight is fractionized into molecular ions having low molecular weight to perform mass spectrometry and constituting molecules were compared.

Herein, a secondary ion mass spectrometry (SIMS) where high energy atomic ion is allowed to collide protein into ionization, an electron desorption ionization (ED) where a molecule is fractionized into molecular ions having low molecular weight by electron impact and mass spectrometry is performed, a matrix-assisted laser desorption ionization (MALDI) and the like, for example, are known as an ion production method in the conventional mass spectrometry of protein.

However, there existed a problem that a mass spectrometer of high resolving power was necessary in order to perform mass spectrometry to polymer ion, a problem that the presence of a fragment ion, which was decomposed and produced in a half-way manner, made mass spectrometry difficult, or the like in all of the above-described methods.

On the other hand, a laser atomization resonance ionization microprobe (LARIMP) method where atomization and ionization are performed by a nano second laser, for example, is known as a mass spectrometry of a sample labeled by an isotopic element in performing chemical analysis.

However, since the LARIMP method requires two laser sources, which are an atomization laser for atomizing labeled elements, and a resonance ionization laser for ionizing the atom of atomized labeled elements, there existed a problem that a system constitution became complicate.

Moreover, since the atom of the labeled element needs to be resonance ionized in the LARIMP method as described above, laser beam of unique wavelength must be irradiated to the atom of each labeled element, and there existed a problem that conducting efficient analysis was extremely difficult in the state where various types of labeled isotopes were mixed.

The present invention has been created in view of the above-described various problems that the conventional art has, and its object is to provide a method of analyzing protein using laser ablation, where atomic ions of constituting atoms, which constitute each type of protein such as protein contained in a specimen taken from a living body, are produced, and the produced atomic ions are analyzed, which is a method of analyzing protein using laser ablation that does not require the use of a spectrometer of high resolving power. More specifically, when performing mass spectrometry to each type of protein such as the protein contained in the specimen taken from the living body, for example, its object is to provide a mass spectrometry of protein using laser ablation where possibility that the analysis of mass spectrum becomes difficult is eliminated and high resolving power is not required in a mass spectrometer.

Further, it is an object of the present invention to provide a method of analyzing protein using laser ablation, which is capable of simultaneously realizing atomization and ionization of the constituting atoms that constitute each type of protein such as the protein contained in the specimen taken from the living body by one laser source, and irradiation control of laser is significantly simplified.

Furthermore, it is an object of the present invention to provide a method of analyzing protein using laser ablation, which is capable of performing efficient analysis even in the state where various types of labeled isotopes are mixed.

### Disclosure of Invention

To achieve the above-described objects, the method of analyzing protein using laser ablation according to the present invention is that each type of protein such as the protein contained in the specimen taken from the living body is ablated by ultra-short pulse laser beams, the protein is atomic ionized to produce atomic ions and the produced atomic ions are analyzed. Thus, it is possible to conduct chemical analysis of each type of protein.

Specifically, according to the method of analyzing protein using laser ablation of the present invention, by ablating each protein such as the protein contained in the specimen taken from the living body by ultra-short pulse laser beams, the protein is decomposed into pieces and atomized by each atom that constitutes the protein, and the atomized atom is simultaneously ionized into a monovalent ion, and quantitative analysis can be performed by analyzing atomic ions produced by the ionization.

Therefore, when performing mass spectrometry in the method of analyzing protein using laser ablation of the present invention, mass spectrometry is conducted to atomic ions having low mass, so that not only the possibility that the analysis of mass spectrum becomes difficult is eliminated but also there is no need to use a mass spectrometer having high resolving power.

Further, as described above, according to the method of analyzing protein using laser ablation of the present invention, by ablating protein by one type of ultra-short pulse laser beams, the protein can be atomized and the ionization of the atomized atoms into the monovalent ion can be simultaneously performed efficiently. Thus, the irradiation control of laser is simplified, and various types of labeled elements can be used simultaneously in chemical analysis, for example, so that analysis efficiency can be improved remarkably.

In other words, since the atomization and ionization of the labeled elements can be simultaneously performed by one type of ultra-short pulse laser beams in the method of analyzing protein using laser ablation of the present invention, an analysis operation can be significantly simplified and the analysis efficiency can be remarkably improved comparing to the conventional methods.

Furthermore, since the above-described ionization is ionization (non-resonance ionization) that is performed through a non-resonance process by high peak value intensity of ultra-short pulse laser beams, each labeled atom can be severally ionized even in the state where various types of labeled isotopes are mixed, by which application to a multi-label system is easy and highly accurate and highly efficient analysis of polymer can be performed.

Specifically, the present invention is a method of analyzing protein using laser ablation where by irradiating laser beams on protein to be analyzed and ablating the protein, the protein is atomized into constituting elements, the atomized constituting elements are ionized, and the ionized constituting elements are analyzed, in which the laser beams that irradiate the protein to be analyzed and ablate the protein are ultra-short pulse laser beams, the ultra-short pulse laser beams are irradiated on a chip having the protein fixed thereon, protein is atomized into constituting elements and ionized simultaneously by ablating the protein fixed on the chip by the ultra-short pulse laser beams, and the ionized constituting elements are analyzed.

Further, the present invention is that the above-described chip having the protein fixed thereon is a chip having particular protein fixed thereon in which the particular protein reacted with and bonded a substance having specific bond to the particular protein fixed on the chip.

Further, the present invention is that the substance having specific bond to the above-described particular protein is a molecule having specific bond with protein.

Furthermore, the present invention is that the molecule having specific bond with the above-described protein is nucleic acid having specific bonding characteristic with protein.

Further, the present invention is that the substance having specific bonding characteristic to the above-described particular protein is protein that exerts a specific bonding action among protein.

Further, the present invention is that the above-described protein that exerts the specific bonding action among the protein is antibody.

Furthermore, the present invention is that the chip having the above-described protein fixed thereon is formed by pouring solution containing protein that reacts with the above-described antibody on a chip having the above-described antibody fixed thereon, allowing protein that reacts with the above-described antibody to react with the above-described antibody, and allowing the protein that reacts with the above-described antibody to bond the above-described antibody.

Further, the present invention is that an element label is attached to the protein fixed on the above-described chip.

Further, the present invention is that the above-described element label is a stable isotopic element.

Further, the present invention is that the above-described element label is labeled by using a puromycin derivative.

Furthermore, the present invention is that the above-described element label is labeled by a sandwich method.

Further, the present invention is that the above-described element label is directly labeled to protein in a sample.

Further, the present invention is that the above-described chip is a multi-channeled chip.

Further, the present invention is that a sample containing protein to be analyzed and labeled protein solution, in which a label is attached to the protein to be analyzed, are mixed and poured on the above-described chip, competitive assay is performed in which a substance having specific bond to particular protein fixed on the above-described chip, the above-described protein to be analyzed, and the above-described labeled protein are bonded competitively, and the particular protein is fixed on the above-described chip.

Furthermore, the present invention is that ultra-short pulse laser beams that are irradiated on the protein to be analyzed and ablate the protein has a pulse time width of 10 pico seconds or less and a peak value output of 10 mega watts or more.

Furthermore, the present invention is that ultra-short pulse laser beams that are irradiated on the protein to be analyzed and ablate the protein has a pulse time width of 1 femto second or more and a peak value output of 1 giga watt or more and 10 giga watts or less.

Further, the present invention is that by moving at least one of the ultra-short pulse laser beams that ablate protein and protein to be analyzed, the ultra-short pulse laser beams that ablate protein ablate the protein to be analyzed without omission and duplication to perform analysis.

Further, the present invention is that the analysis of the above-described ionized constituting elements is mass spectrometry by a time-of-flight method.

Further, the present invention is that substances that need to be fixed on the above-described chip are fixed as a mixture, solution attached with a different label for a substance to be measured is allowed to react with the mixture, and plural types of substances are detected from the mixture.

Still further, the present invention is that a sample is fixed on the above-described chip, antibody to a measuring subject, which has been labeled in plural types, is poured to measure a plurality of substances.

Herein, when ablating protein by the ultra-short pulse laser beams in the present invention, irradiating one shot (one pulse) of the ultra-short pulse laser beams on protein is enough. However, plural shots (plural pulses) of the ultra-short pulse laser beams may be irradiated on protein and the number of shots (pulse number) of the ultra-short pulse laser beams to be irradiated on protein should be appropriately selected.

Further, it is preferable that the ultra-short pulse laser beams be one having the pulse time width of 10 pico seconds or less. For example, it is appropriate to use femto-second laser beams irradiated from a laser that is generally called a femto-second laser having the pulse time width of 1 femto second or more and 1 pico second or less.

Furthermore, 10 mega watts or more is preferable as the peak value output from the ultra-short pulse laser beams, and more particularly, 1 giga watt or more and 10 giga watts or less is preferable.

This is because there is a possibility that multivalent ions are produced to make the analysis of mass spectrum difficult when the peak value output from the ultra-short pulse laser beams is 10 giga watts or larger, and the efficiency of atomization and ionization is reduced to make it difficult to observe atomic ion signals when the peak value output from the ultra-short pulse laser beams is 10 mega watts or less.

It is to be noted that, according to experiments, which are described later, conducted by the inventors of the present invention, extremely good results could be obtained when the pulse time width was 110 femto seconds and the peak value output was 2 giga watts.

Further, according to the present invention, the ultra-short pulse laser beams such as the femto-second laser beams capable of simultaneously performing atomization and ionization efficiently are irradiated on a protein sample that is labeled by an isotopic element or the like. Therefore, it is not necessary to selectively ionize the labeled elements and various types of labeled elements can be used. Moreover, since the repetition rate of laser irradiation can be increased to several kHz, the invention is suitable for high-speed analysis.

Furthermore, in the present invention, by moving at least one of the ultra-short pulse laser beams that ablate each type of protein to be analyzed such as the protein contained in the specimen taken from the living body and the protein to be analyzed, the ultra-short pulse laser beams that ablate protein ablate the protein to be analyzed without omission and duplication to perform analysis. Specifically, in the present invention, by the movement of a spot of short pulse laser beams and the chip having protein to be analyzed fixed thereon as a sample, ablation of a large number of protein samples fixed on a wide area can be performed without omission and duplication. This makes it possible to use a chip where protein is fixed by antigen-antibody reaction as a chip having antigen to protein to be analyzed fixed thereon, which is particularly effective.

According to the present invention, analysis speed becomes significantly faster than conventional one due to this characteristic.

Herein, a specific application example of the present invention is the analysis of protein using the chip where protein is fixed by antigen-antibody reaction as a chip having antigen to protein to be analyzed fixed thereon, for example, and it is possible to increase the speed of the analysis.

On such occasion, an element label can be used as a label according to the present invention. More specifically, various types of isotopic elements can be used as the element label, and when stable isotopic elements are used as the element label, for example, it is possible to increase the variety of labels to the number (270 types) of various types of stable isotopes. This means that the amount of information can be increased dramatically comparing to a fluorescence method (2 to 6 types) that is a conventional labeling method and radioactive isotopic elements (about 10 types).

More specifically, it is possible to use protein by labeling with a stable isotope such as ³⁹K and ⁴¹K that are group 1 stable isotopes in the periodic table, ³²S and ³⁵S that are group 16 stable isotopes in the periodic table, ³⁵ Cl and ³⁷Cl that are group 17 stable isotopes in the periodic table, or ¹¹⁸Sn and ¹²⁰Sn that are stable isotopes of transition metal in the periodic table, as a label used in the experiment of protein analysis using the chip where protein is fixed by antigen-antibody reaction as a chip having antigen to protein to be analyzed fixed thereon.

After protein is allowed to bond protein that is a target on the above-described chip, it is ablated by ultra-short pulse laser beams to perform atomic ionization of molecules. After that, the mass spectrometer detects mass, and thus the quantity of isotopic element contained in the bonded protein can be determined. Therefore, the target protein can be analyzed by calculating the ratio of volume in labeled molecules.

Herein, in comparison with labels currently used, the variety of labels can be increased to as many as 270 types when stable isotopic elements are used, for example.

Moreover, when a plurality (three types or more) of protein labeled by separate elements are mixed and a multi-channeled chip that were allowed to bond and react with a target at the same time, data between a plurality of samples can be compared.

As described, the present invention can establish high-sensitive and high-speed mass spectrometry by various types of stable isotopic element labels, and therefore, the present invention can be applied for all research fields where labeling is performed by fluorochrome or radioactive isotopes.

Furthermore, according to the present invention, stable isotopic elements can be used as the labeled element without using the radioactive isotopic elements, so that facility to be used in such case is not restricted and installation in medical facility and private firms is possible.

Additionally, in the present invention, regarding multi-channeling where a plurality of labels are used at a time, when the substances fixed on the chip are not spotted by types like a micro array, but fixed as a mixture and solution attached with a different label by each substance that needs to be measured is allowed to react with the mixture, and thus it is possible to detect plural types of substances from one spot.

With this method, labor for fabricating the micro array can be omitted drastically, and a custom-made substrate for adjusting solution containing substances that a user wants to measure can be easily formed.

Furthermore, the sample can be contrarily mounted on the substrate. For example, a plurality of substances can be measured when antibody to a measuring subject, which is labeled by various types, is poured.

### Brief Description of the Drawings

Fig. 1 is a conceptual constitution exemplary view of a constitution example of a mass spectrometric system for analyzing the mass of protein, which is an example of an analysis system that can be used when implementing a method of analyzing protein using laser ablation according to the present invention.
Fig. 2 is an exemplary view showing an example of the method of analyzing protein using laser ablation according to the present invention.
Fig. 3 is an exemplary view for explaining a sandwich method using antibody as a method of labeling protein that needs to be measured.
Fig. 4 is an exemplary view of a chip being a target used in experiment 1 conducted by the inventors of the present invention.
Fig. 5 shows the experiment result of experiment 1 conducted by the inventors of the present invention, and is a graph showing mass spectrum by ablation on spot 1 and spot 2, which were measured by a quadruple mass spectrometer.
Fig. 6 is a graph showing only the mass spectrum measured by ablation on spot 2 which is extracted from Fig. 5.
Fig. 7 is an exemplary view of a chip being a target used in experiment 2 conducted by the inventors of the present invention.
Fig. 8 is an exemplary view showing the corresponding relation between chip 1 and chip 2 which were used in experiment 2, and measurement results.
Fig. 9 is a graph of TOF spectrum that was measured by ablation on spot 1 of chip 1.
Fig. 10 is a graph of TOF spectrum that was measured by ablation on spot 2 of chip 1.
Fig. 11 is a graph of TOF spectrum that was measured by ablation on spot 1 of chip 2.
Fig. 12 is a graph of TOF spectrum that was measured by ablation on spot 2 of chip 2.
Fig. 13 is a graph of TOF spectrum that was measured by ablation when sample concentration in experiment 3 was 0.2mg/ml.
Fig. 14 is a table showing the detected quantity of samarium (Sm) that was calculated from TOF spectrum.
Fig. 15 is a graph showing the relation between the sample concentration and the quantity of Sm detected.

### Explanation of Reference Numerals

- 10: Mass spectrometric system
- 12: Vacuum tank
- 14: Target
- 16: Quadruple mass spectrometer
- 18: Rotational inlet terminal
- 20: Ultra-short pulse laser
- 22: Focus lens
- 100: Chip
- 102: Antibody
- 104: Labeled protein
- 106: Sample
- 108: Target protein contained in sample
- 200: Chip
- 202: Antibody
- 206: Sample
- 208: Target protein contained in sample
- 210: Labeled element
- 212: Antibody (labeled antibody)
- 1000: Substrate
- 2000: Substrate

### Best Mode for Implementing the Invention

In the following, an example of embodiments of the method of analyzing protein using laser ablation according to the present invention will be described in detail referring to the attached drawings.

According to the method of analyzing protein using laser ablation, each type of protein such as protein contained in a specimen taken from a living body can be analyzed.

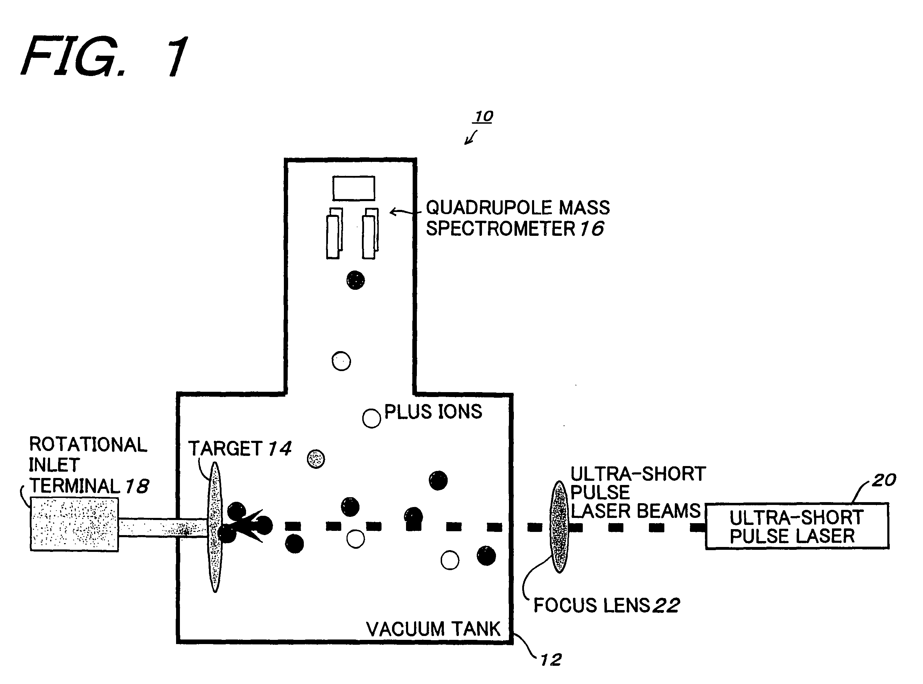

Fig. 1 shows the conceptual constitution exemplary view of a constitution example of the mass spectrometric system for analyzing the mass of protein as an example of the analysis system that can be used when implementing the method of analyzing protein using laser ablation according to the present invention.

The mass spectrometric system 10 has a vacuum tank 12 that can be set to the vacuum level of 10⁻⁸ to 10⁻⁶ Torr, for example, a target 14 arranged in the vacuum tank 12, a quadruple mass spectrometer 16 as a mass spectrometer arranged in the vacuum tank, a rotational inlet terminal 18 that rotates the target 14, a ultra-short pulse laser 20 that emits ultra-short pulse laser beams such as the femto-second laser beam, for example, to be irradiated on the target 14, and a focus lens 22 that focuses the ultra-short pulse laser beams irradiated from the ultra-short pulse laser 20 on the target 14.

Herein, as the ultra-short pulse laser 20, one capable of irradiating ultra-short pulse laser beams having the pulse time width of 1 femto second or more and 1 pico second or less and the peak value output of 10 giga watts or less can be used.

More specifically, one that is made up of a titanium-sapphire laser and includes parameters shown below, for example, can be used as such ultra-short pulse laser 20.

Peak width (pulse time width) : No more than 100 fs (femto second)
Output: 50 to 480µJ (micro joule)
(Peak value output: 0.5 to 4GW (giga watt))
Wavelength: No more than 800nm (nano meter)
Repetition: 1kHz (kilo hertz)

Further, the quadruple mass spectrometer 16 is installed in a vertical direction by 90 degrees with respect to the irradiation direction of the ultra-short pulse laser beams that are emitted from the ultra-short pulse laser 20 and irradiated on the target 14.

Meanwhile, it goes without saying that a time-of-flight mass spectrometer (TOF MASS) may be used as the mass spectrometer instead of the above-described quadruple mass spectrometer 16.

Furthermore, the focal length of the focus lens 22 that focuses the ultra-short pulse laser beams emitted from the ultra-short pulse laser 20 is set to 25cm, for example.

In the above-described constitution, description will be made for a method of performing mass spectrometry using the above-described mass spectrometric system 10 by the method of analyzing protein using laser ablation according to the present invention.

Herein, the method of analyzing protein using laser ablation according to the present invention is that the sample of each type of protein such as the protein contained in the specimen taken from the living body is detected and analyzed by using ablation by the ultra-short pulse laser beams emitted from the ultra-short pulse laser 20 such as the femto-second laser and an analyzer such as the mass spectrometer such as the quadruple mass spectrometer 16 and the time-of-flight mass spectrometer.

Specifically, the method of analyzing protein using laser ablation according to the present invention is a method of analyzing protein using laser ablation, where laser beams are irradiated on protein to be analyzed to ablate the protein, the protein is atomized into constituting elements, the atomized constituting elements are ionized, and the ionized constituting elements are analyzed, and the laser beams ablating the protein is the ultra-short pulse laser beams where the ultra-short pulse laser beams are irradiated on the chip having protein fixed thereon, the ultra-short pulse laser beams ablate the protein fixed on the chip to simultaneously atomize and ionize the protein into the constituting elements, and thus analyze the ionized constituting elements.

Specifically, the chip having protein fixed thereon is arranged in the vacuum tank 12 as the target 14, the ultra-short pulse laser beams such as the femto-second laser beam emitted from the ultra-short pulse laser 20 are irradiated on the chip being the target 14 to perform ablation, and analysis is conducted by the mass spectrometer such as the quadruple mass spectrometer 16 and the time-of-flight mass spectrometer.

In such occasion, when the protein to be analyzed is attached with a label by an element label or the like, the chip having the labeled protein fixed thereon is arranged as the target 14 in the vacuum tank 12, the ultra-short pulse laser beams such as the femto-second laser beam emitted from the ultra-short pulse laser 20 are irradiated on the chip being the target 14 to perform ablation, the labeled elements are measured by the mass spectrometer such as the quadruple mass spectrometer 16 and the time-of-flight mass spectrometer, and thus the protein to be analyzed can be detected and analyzed.

Herein, as the chip having protein fixed thereon, a chip having protein fixed thereon, where the protein reacted with and bonded a substance (such as antibody) having specific bond to particular protein fixed on the chip and the protein is fixed on the chip, can be used. Specifically, a chip where protein has been fixed by antigen-antibody reaction on a chip, on which antigen to protein to be analyzed is fixed, can be used as the chip having protein fixed thereon, for example.

Therefore, the chip having the labeled protein fixed thereon can be formed as follows, for example.

Specifically, solution of labeled protein being a sample is poured on the chip on which a substance (such as antibody) having specific bond to particular protein has been fixed, the labeled protein and the above-described substance having specific bond are allowed to react, and particular protein in the labeled protein is fixed on the chip. Furthermore, by cleaning the chip on which the particular protein out of the labeled protein is fixed, a chip having the labeled protein fixed thereon is obtained.

Herein, similar to a DNA chip, by using a chip where various types of substances (such as antibody) severally having specific bond with particular protein are fixed on one chip and by using the chip on which protein severally bonded the various types of substances (such as antibody) having specific bond has been fixed, measurement of various types of protein can be performed simultaneously.

Further, as the substance having specific bond to particular protein fixed on a chip, it is possible to use a molecule having specific bond to protein or, furthermore, protein such as antigen-antibody that exerts a specific bonding action among protein.

It is to be noted that a nucleus acid called as an aptamer having specific bond similar to antigen can be used as the molecule having specific bond to protein, for example. Aptamer can be formed by a method called SELEX by which one having high bonding characteristic with a target substance such as protein from the pool of oligonucleotide that has been randomly synthesized.

Further, as the protein such as antigen-antibody that exerts the specific bonding action among protein, protein that forms a receptor or a complex with ligand, which are known to cause specific bond in a living body can be used.

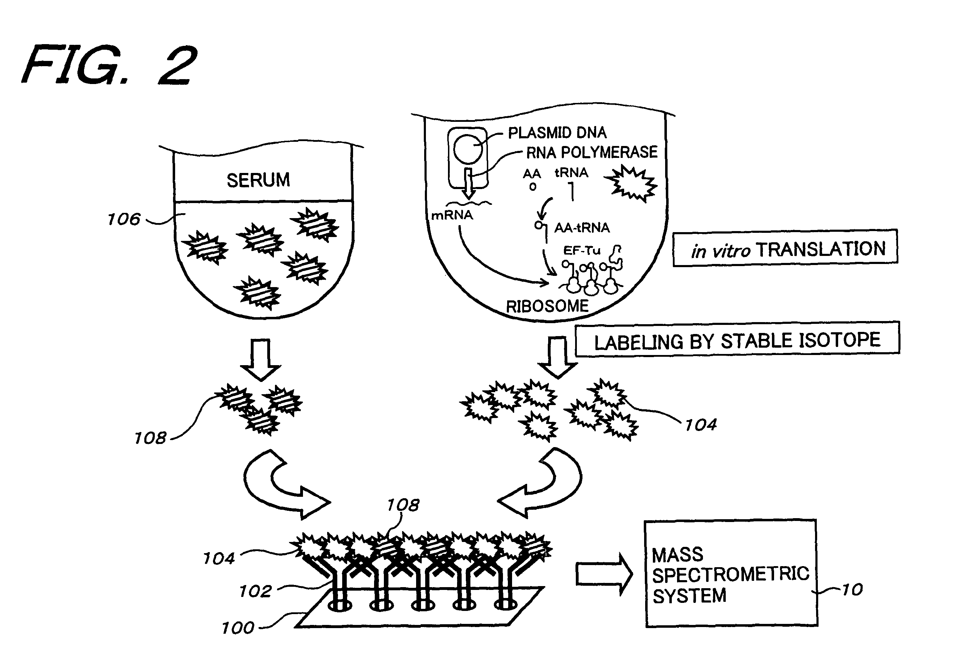

Herein, an example of the method of analyzing protein using laser ablation according to the present invention will be described in more details referring to Fig. 2.

Specifically, in the above-described method of analyzing protein using laser ablation according to the present invention, a chip 100, on which antibody 102 to protein that needs to be detected is fixed, is formed.

Next, the protein that needs to be detected is synthesized by in vitro translation or the like, and labeled by an element that is not contained in the protein (stable isotopic element such as Se, Eu, Sm, Tb and Fe) to form labeled protein 104.

Herein, a selemethionine method, an in vitro virus method using puromycin derivatives, or the like, for example, can be used as a labeling method.

Meanwhile, although labeling by fluorochrome is generally used in the in vitro virus method, an element labeling body is synthesized and element labeling can be performed in a similar method to the case using fluorochrome.

Next, several types of solution having different concentration of synthesized labeled protein are measured to take their calibration curve.

Next, a sample 106 (such as serum, for example) taken from the living body is mixed with the labeled protein solution whose concentration has been adjusted, the mixture is poured onto the chip 100 to perform competitive assay, and competitive assay is performed by antigen-antibody reaction in which the antibody 102, the labeled protein 104, and target protein 108 contained in the sample 106 are allowed to bond competitively. Then, after the chip 100, on which the antibody 102, the labeled protein 104, and the target protein 108 contained in the sample 106 have been bonded and fixed, is cleaned and dried, the femto-second laser beam from the ultra-short pulse laser 20 is irradiated on the chip having the target protein 108 fixed thereon as the target 14 to perform ablation, a quantity that the labeled protein 104 has reacted with the antibody 102 is measured by measuring the labeled elements by using the quadruple mass spectrometer 16 or the time-of-flight mass spectrometer, and thus the concentration of the target protein 108 contained in the sample 106 is detected and analyzed.

Herein, since there is a possibility to cause difference in the calibration curve due to the status of the chip, it is desirable to create the calibration curve and measure the sample simultaneously on a same chip.

Specifically, to solve such problems, the chip on which the antibody, the labeled protein, and the target protein contained in the sample are bonded should be multi-channeled, and the multi-channeled chip can further improve the accuracy of the above-described method according to the present invention.

The multi-channeling can be realized by the method explained below, for example.

In other words, since there is a possibility to cause difference in the calibration curve due to the status of the chip, it is desirable to create the calibration curve and measure the sample simultaneously on a same chip in order to prevent the difference. Therefore, when measuring protein called A, solution of labeled protein, to which different labels (for example, labeling by several types of Fe isotope) are attached by each concentration, is prepared. When it is mixed with the sample and allowed to react with the chip having antibody fixed thereon, the creation of the calibration curve and the sample measurement can be performed simultaneously.

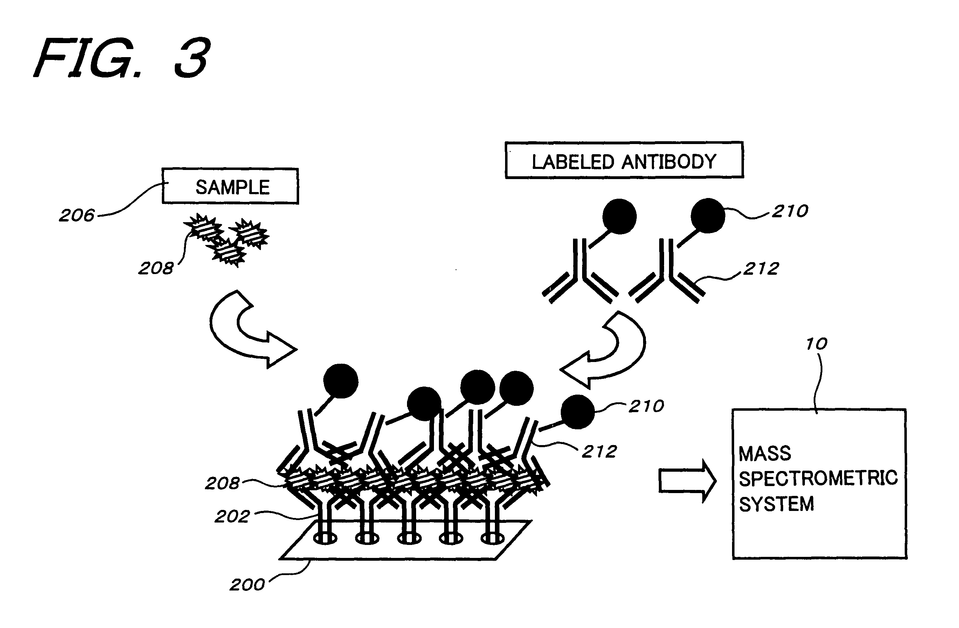

Next, as the method of labeling protein that needs to be measured, a sandwich method using antibody may be used other than the above-described method where the labeled protein is previously synthesized before mixing with the sample. The sandwich method will be described referring to Fig. 3.

In the sandwich method, a sample 206 is allowed to react with a chip 200 on which antibody 202 to target protein that needs to be detected is fixed, and target protein 208 contained in the sample 206 and the antibody 202 are bonded.

Next, antibody (labeled antibody) 212 labeled by a labeled element 210 is poured from above the target protein 208 contained in the sample 206 that bonded the antibody 202, and the target protein 208 contained in the sample 206 is allowed to react with the labeled antibody 212 and bonded.

Then, after the chip 200, on which the antibody 202, the protein 208, and the labeled antibody 212 labeled by the labeled element 210 have been bonded, is cleaned and dried, the femto-second laser beam from the ultra-short pulse laser 20 is irradiated on the chip 200 as the target 14 to perform ablation, the labeled element 210 is measured by the quadruple mass spectrometer 16 or the time-of-flight mass spectrometer, and thus the concentration of the target protein 208 contained in the sample 206 is detected and analyzed.

Further, the sandwich method is not limited to the above-described one, but a different producing animal of the upper antibody when sandwiching and pinching the target protein that needs to be detected is selected, and labeled secondary antibody may be poured from above to perform detection, for example.

Furthermore, a method of directly labeling the protein in the sample may be used as the method of labeling protein that needs to be measured.

Specifically, the protein contained in the sample may be labeled by iodine, for example. An iodo-bead method (trademark of Pierce Chemical Co.) can be used, for example, to perform the method of labeling the protein by iodine.

Then, when using a sample where the protein in the sample has been directly labeled, the sample is poured onto the chip on which antibody to the target protein that needs to be detected is fixed, the target protein contained in the sample, which needs to be detected, is allowed to react with the antibody, and the target protein contained in the sample, which needs to be detected, and the antibody are bonded by antigen-antibody reaction.

Next, after the chip, on which the target protein contained in the sample, which needs to be detected, and the antibody have been bonded, is cleaned and dried, the femto-second laser beam from the ultra-short pulse laser 20 is irradiated on the chip as the target 14 to perform ablation, the labeled element is measured by the quadruple mass spectrometer 16 or the time-of-flight mass spectrometer, and thus the concentration of the target protein contained in the sample, which needs to be detected, is detected and analyzed.

In the following, description will be made for the experiment results of the mass spectrometry conducted by the inventors of the present invention by using the above-described mass spectrometric system 10.

### (1) Experiment 1

### (1-1) Forming of chip

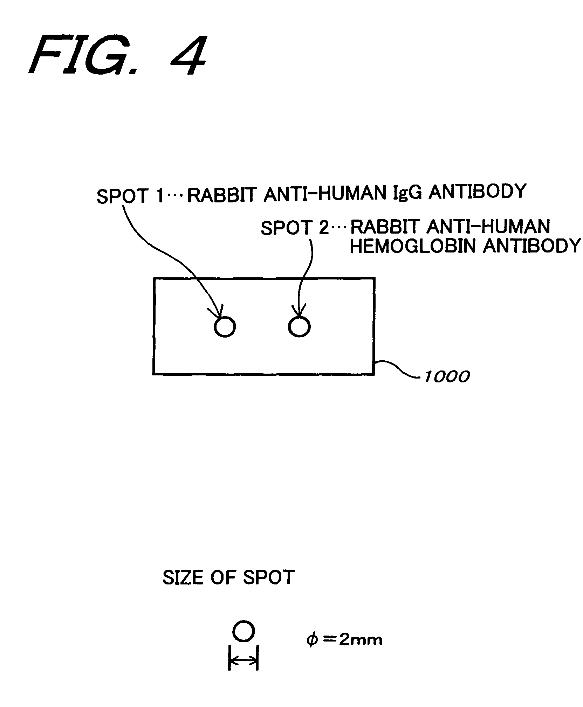

Firstly, the forming of a chip being the target 14 will be described referring to Fig. 4. The chip is formed as follows.

Specifically, poly-L-lysine-coated slide glass was used as a substrate 1000. The poly-L-lysine-coated slide glass was formed in such a manner that slide glass was immersed in 3% poly-L-lysine solution for 1 hour after NaOH treatment, and dried at 80°C after cleaning by water. Next, rabbit anti-human hemoglobin antibody (manufactured by Sigma Co., Ltd.) and rabbit anti-human IgG antibody were severally decomposed in PBS at the concentration of 0.2mg/ml, and were spotted on the poly-L-lysine coated slide glass (refer to Fig. 4: spot 1 is the rabbit anti-human IgG antibody and spot 2 is the rabbit anti-human hemoglobin antibody). Then, after removing extra antibody by PBS solution containing 3% non-fat milk, and 0.1% Tween-20, the glass was immersed in blocking solution containing 3% non-fat milk and 0.02% sodium azide and left to stand for one night at 4°C. In addition, the glass was cleaned by PBS to remove blocking solution after 10 minutes of centrifuging at 10,000xg, and a chip having antibody fixed thereon was formed.

Herein, the material of the substrate does not need to be glass, and it may be metal or insulator. A substrate having higher heat conduction causes higher ion detection efficiency in the laser ablation using ultra-short pulse laser beams. It is to be noted that a solid substance is used as the substrate, and it is preferable that the heat conductivity of the solid substance used as the substrate be 0.1W•m-1•K-1 or more.

### (1-2) Adjustment and reaction of protein solution

Next, the adjustment of protein solution and the reaction with the antibody fixed on the chip are performed. The adjustment and reaction of protein solution is conducted as follows.

Human hemoglobin (manufactured by Wako Pure Chemical Industries, Ltd.) was dissolved in PBS at the concentration of 2mg/ml, and the protein solution was adjusted. Extra PBS was shaken off from the chip having antibody fixed thereon, the protein solution was immediately placed on a chip surface on which antibody was fixed, cover glass was gently covered from above, and it was left to stand for 2 hours at 4°C. After that, the chip was immersed in PBS to remove the cover glass and protein solution, treated in 0. 005M Tris-HCl, 0.005% Tween20, and pH7. 8, dried after subsequent treatment by PBS, and a chip being the target 14 was obtained.

### (1-3) Measurement

The target 14 formed as described above is installed in the vacuum tank 12, and the inside of the vacuum tank is drawn vacuum to set the degree of vacuum inside the vacuum tank 12 to 10⁻⁶ Torr or less.

Next, ultra-short pulse laser beams emitted from the ultra-short pulse laser 20 are focused on the target 14 by using the focus lens 22 to ablate spot 1 or spot 2 formed on the target 14.

It is to be noted that the pulse width of the ultra-short pulse laser beams emitted from the ultra-short pulse laser 20 is 110 femto seconds and its output is 230µJ.

Then, the mass of monovalent ion generated by the irradiation of ultra-short pulse laser beams onto the target 14 was measured by the quadruple mass spectrometer 16.

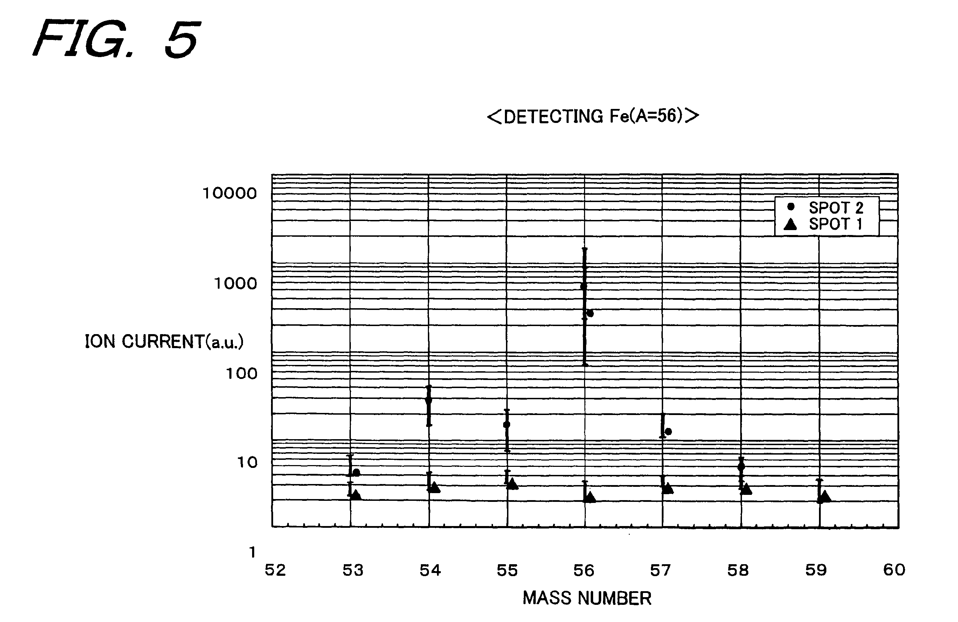

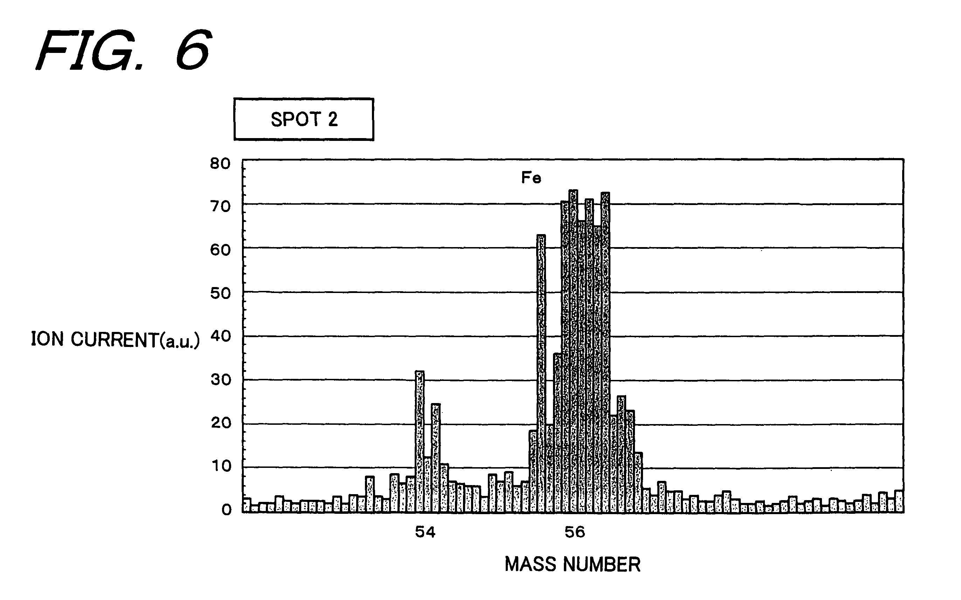

Fig. 5 shows the mass spectrum caused by the ablation to spot 1 and spot 2, which were measured by the quadruple mass spectrometer 16 using the above-described method. In addition, Fig. 6 shows by extraction only the mass spectrum measured by the ablation on spot 2 shown in Fig. 5.

As it is clear from the experiment results shown in Fig. 5 and Fig. 6, Fe was detected from spot 2, and hemoglobin could be detected by the method of the present invention.

Meanwhile, an element label was not attached to the protein to be detected in the above-described experiment 1, but it is a matter of course that protein attached with the element label can be analyzed as shown in experiment 2 and experiment 3. In the following, experiment 2 and experiment 3 will be described.

### (2) Experiment 2

### (2-1) Forming of chip

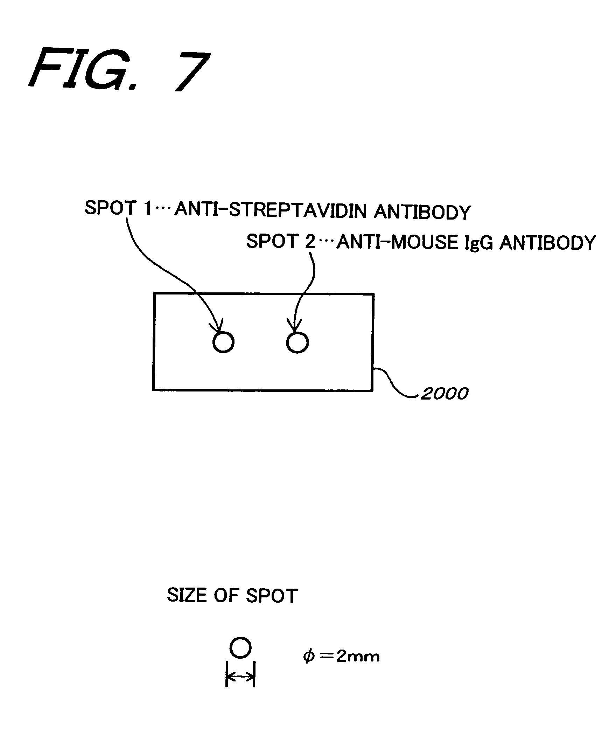

Firstly, the forming of a chip being the target 14 will be described referring to Fig. 7. The chip is formed as follows.

Specifically, poly-L-lysine-coated slide glass was used as a substrate 2000. The poly-L-lysine-coated slide glass was formed in such a manner that slide glass was immersed in 3% poly-L-lysine PBS solution for 1 hour after NaOH treatment, and dried after cleaning by water for 15 minutes. Next, PBS in which anti-streptavidin antibody (manufactured by Cortex Biochem Inc.) and anti-mouse IgG antibody (manufactured by Southern Biotechnology Associates Inc.) were severally dissolved at the concentration of 0 .1mg/ml was severally spotted by 3µl/spot on the poly-L-lysine coated slide glass that was formed as described above as shown in Fig. 7, and was naturally dried (in Fig. 7, spot 1 is the anti-streptavidin antibody and spot 2 is the anti-mouse IgG antibody). Then, the dried poly-L-lysine coated slide glass was immersed in Tris-HCl solution (pH7.8) containing 1.5%BSA, 0.1% sodium azide, and 0.05%Tween40 for 15 minutes for cleaning, and subsequently, the glass was immersed in Tris-HCl solution (pH7. 8) containing 1.5%BSA and 0.1% sodium azide, and left to stand for one night at 4°C. The glass was cleaned by PBS to remove blocking solution after 10 minutes of centrifuging at 10,000xg.

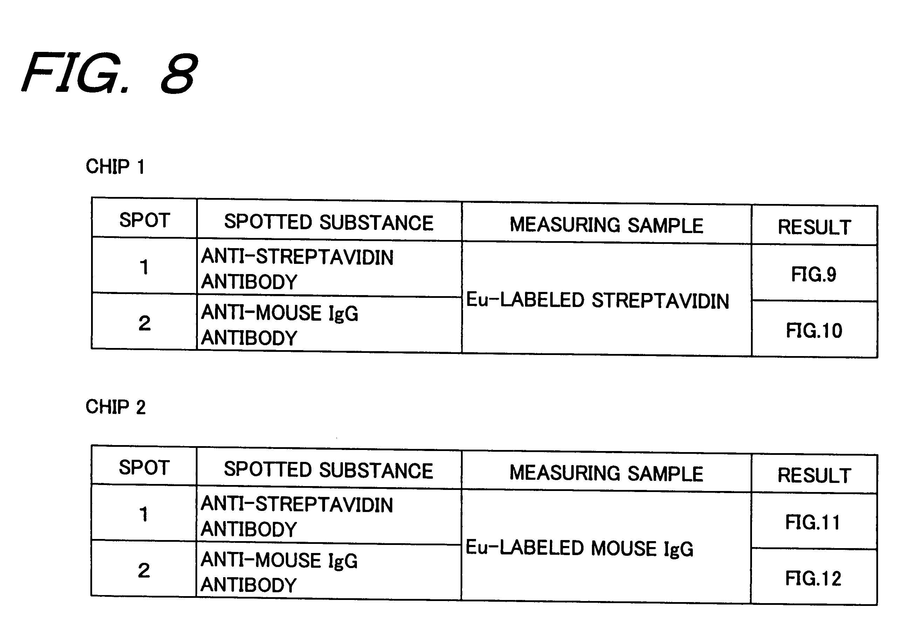

It is to be noted that two pieces of the chips formed as described above were prepared (refer to Fig. 8: chip 1 and chip 2) .

### (2-2) Adjustment and reaction of protein solution

Streptavidin (manufactured by PerkinElmer Inc.) labeled by europium (Eu) was dissolved in Tris-HCl solution (pH7.8) at 0.2mg/ml, it was dropped on spot 1 and spot 2 of chip 1 by 3µl, and the chip was left to stand in room temperature for 2 hours. After that, chip 1 was cleaned by Tris-HCl solution (pH7.8) containing 0.1%Tween20 for 30 minutes, and dried naturally (refer to Fig. 8).

Further, mouse IgG (manufactured by PerkinElmer Inc.) labeled by europium (Eu) was dissolved in Tris-HCl solution (pH7 . 8) at 0. 2mg/ml, it was dropped on spot 1 and spot 2 of chip 2 by 3µl, and the chip was left to stand in room temperature for 2 hours. After that, chip 2 was cleaned by Tris-HCl solution (pH7.8) containing 0.1%Tween20 for 30 minutes, and dried naturally (refer to Fig. 8).

### (2-3) Measurement

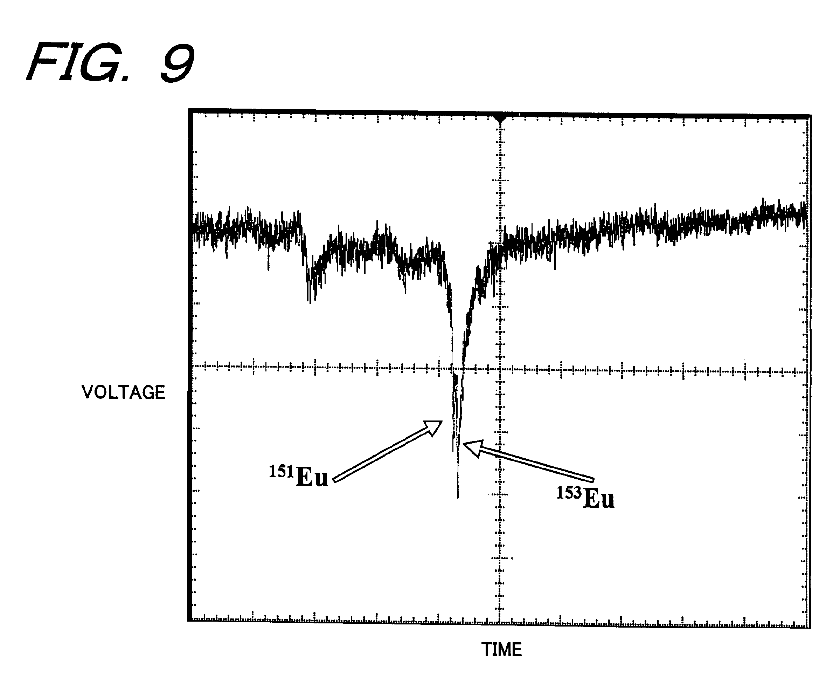

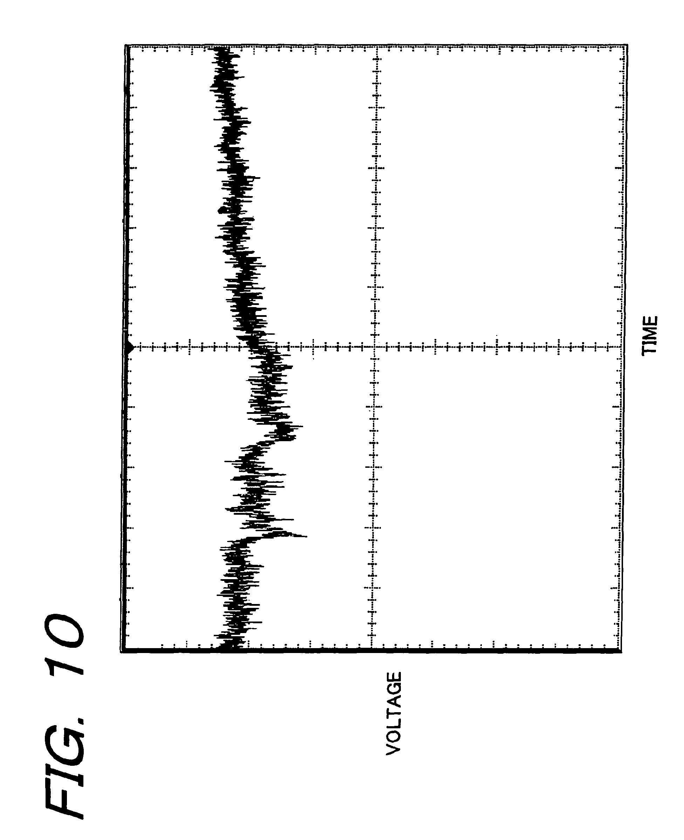

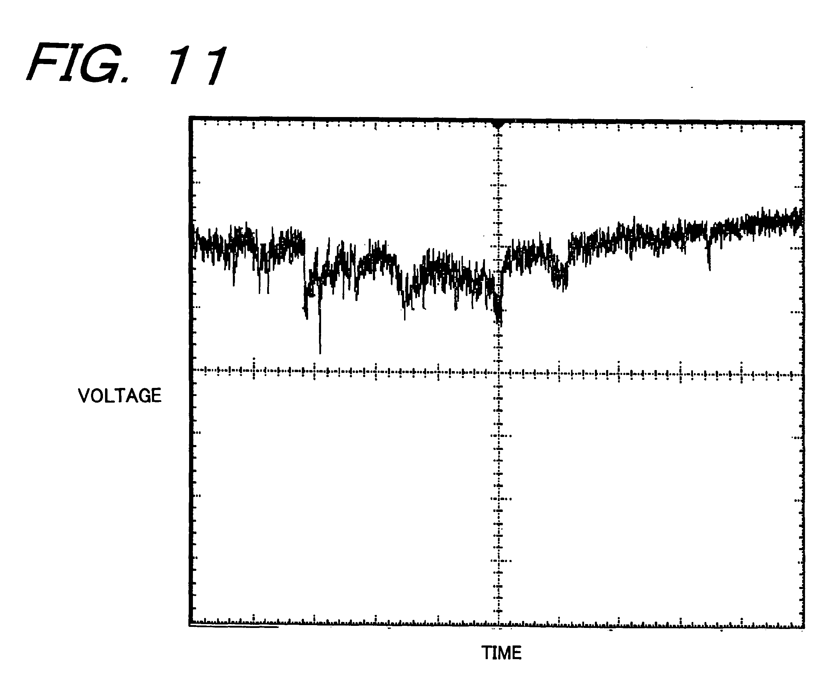

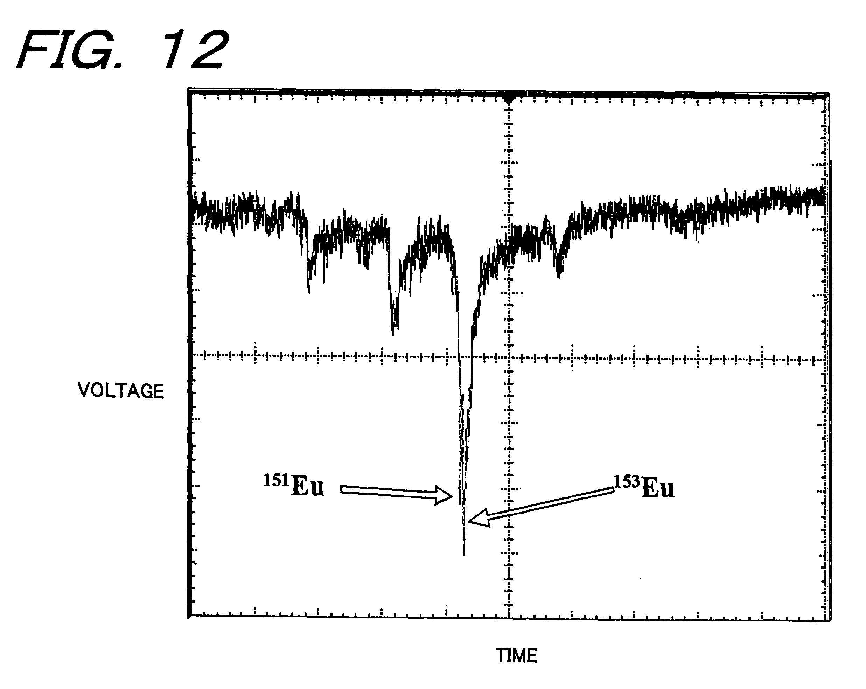

Although the measuring method is the same as the case of experiment 1, the experiment is different from the measuring method of experiment 1 on the point that the output of ultra-short pulse laser beams emitted from the ultra-short pulse laser 20 was set to 120µJ and the mass measurement was conducted using the time-of-flight mass spectrometer. Fig. 9 to Fig. 12 show the experiment results of experiment 2. Herein, Fig. 9 shows the measurement result of spot 1 on chip 1, Fig. 10 shows the measurement result of spot 2 on chip 1, Fig. 11 shows the measurement result of spot 1 on chip 2, and Fig. 12 shows the measurement result of spot 2 on chip 2.

From the experiment results shown in Fig. 9 to Fig. 12, it was made clear that protein contained in the sample could be detected by using a label attached to the protein.

### (3) Experiment 3

A chip was fabricated by the same method as experiment 2, it was allowed to react with measuring protein solution whose concentration was changed, and quantitativeness was studied.

Herein, the adjustment of protein solution was performed as follows. Specifically, Tris-HCl solution (pH7.8) labeled by samarium (Sm), which contains streptavidin (manufactured by PerkinElmer Inc.) at 0.002mg/ml, 0.02mg/ml, 0.1mg/ml, 0.2mg/ml, and 0.5mg/ml, was adjusted, and it was allowed to react with a chip on which anti-streptavidin antibody was spotted in the same conditions as experiment 2 to perform measurement.

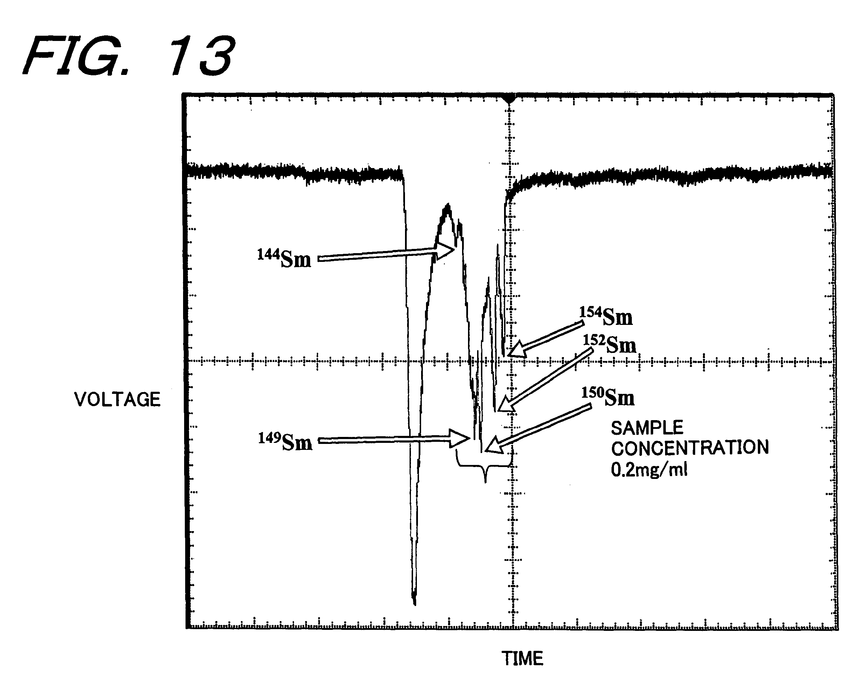

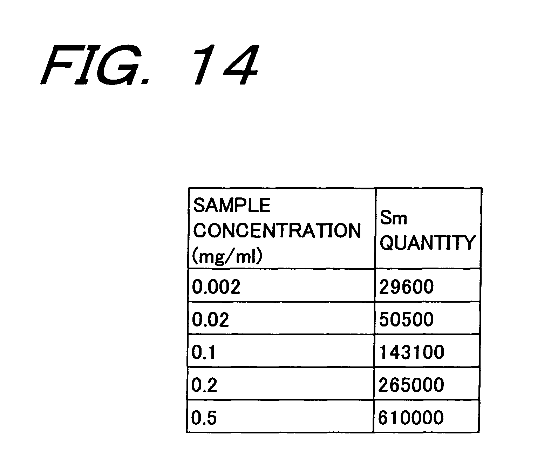

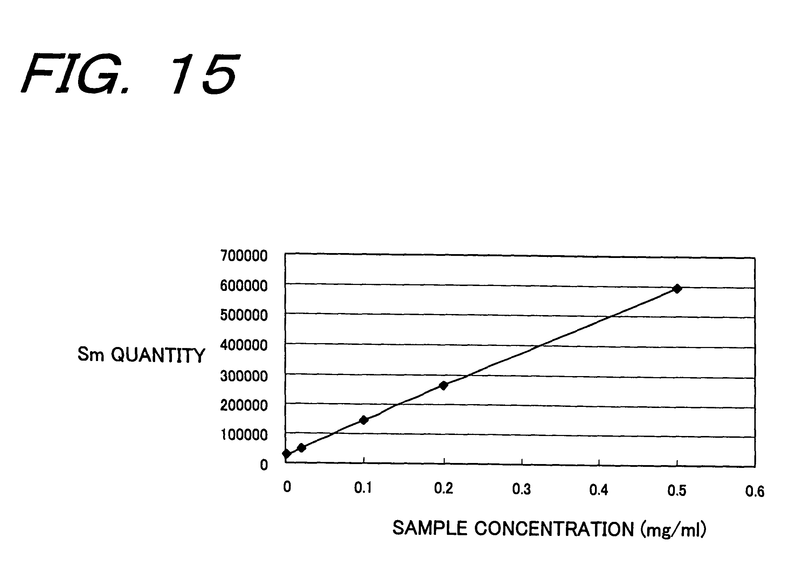

Fig. 13 shows the measurement result when the sample concentration was 0.2mg/ml, and Fig. 14 shows the detected quantity of Sm that was calculated from the mass spectrum. These results made clear, as shown in Fig. 15, that the quantity of measuring protein contained in the sample could be measured quantitatively by using the method according to the present invention.

According to the present invention, with the ablation by ultra-short pulse laser beams on protein that is labeled by a single or a plurality of isotopic element (s), the constituting elements are completely atomic ionized, and the quantitative measurement of protein can be performed by conducting mass spectrometry to the ionized labeled element. This makes it possible to use various types of isotopic elements as labels. Therefore, an applicable range of protein to which mass spectrometry is performed can be broaden remarkably.

In short, protein itself, which has been labeled by isotopic elements, is ionized on an atomic level and thus the labeled element can be detected, so that it becomes possible to remarkably broaden the applicable range where mass spectrometry can be performed. For example, isotopic elements can be used as the label, and the variety of labels can be increased to as many as 270 types that are the number of the stable isotopic elements, for example. This means that the amount of information can be increased dramatically comparing to the fluorescence method (2 types) that is the conventional labeling method and the radioactive isotopic elements (about 10 types).

Meanwhile, the quadruple mass spectrometer was used as the mass spectrometer in the above-described embodiment, but it goes without saying that the invention is not limited to this. The time-of-flight mass spectrometer that performs mass spectrometry by measuring the time of flight of atoms as described above, and in such a case, mass spectrometry of a plurality of atoms can be performed simultaneously in one laser irradiation. Furthermore, it is also possible to perform mass spectrometry of a plurality of atoms when using an ion cyclotron Fourier transform mass spectrometer as the mass spectrometer.

Furthermore, description has been made for mass spectrometry as the method of analyzing protein in the above-described embodiment, but it goes without saying that the invention is not limited to this and the present invention may be used for analysis other than mass spectrometry.

Still further, the rotational inlet terminal 18 for rotating the target 14 was used in the above-described embodiment, but it goes without saying that the invention is not limited to this and appropriate means such as a freely movable table capable of mounting the target 14 thereon may be used.

Further, in the above-described embodiment, the rotational inlet terminal 18 was used to rotate the target 14 to ablate the target 14 without omission and duplication, but it goes without saying that the invention is not limited to this and moving means that moves the irradiation position of ultra-short pulse laser beams on the target may be provided to ablate the target 14 without omission and duplication.

In addition, in the present invention, regarding the multi-channeling where a plurality of labels are used at a time, it is possible to detect a plural types of substances from one spot when the substances fixed on the chip are not spotted by types like the micro array, but fixed as the mixture and solution attached with a different label by each substance to be measured is allowed to react with the mixture.

With this method, labor for fabricating the micro array can be omitted drastically, and a custom-made substrate for adjusting solution containing substances that the user wants to measure can be easily formed.

Furthermore, the sample can be contrarily mounted on the substrate. For example, a plurality of substances can be measured when antibody to a measuring subject, which is labeled by various types, is poured.

### Industrial Applicability

Since the present invention is constituted as described above, it exerts excellent effect that it can provide the method of analyzing protein using laser ablation, in which atomic ions of constituting atoms that constitute protein are produced and the produced atomic ions are analyzed, which is a method of analyzing protein using laser ablation that does not require a mass spectrometer of high resolving power. Herein, in more details, the invention exerts excellent effect that it can eliminate the possibility that the analysis of mass spectrum becomes difficult and does not require the mass spectrometer of high resolving power when performing mass spectrometry, for example.

Further, since the present invention is constituted as described above, it exerts excellent effect that it can drastically simplify the system constitution.

Still further since the present invention is constituted as described above, it exerts excellent effect that it can perform effective analysis even under the state where various types of labeled isotopes are mixed.

## Claims

1. A method of analyzing protein using laser ablation in which by irradiating laser beams on protein to be analyzed and ablating the protein, the protein is atomized into constituting elements, the atomized constituting elements are ionized, and the ionized constituting elements are analyzed, wherein
the laser beams that irradiate the protein to be analyzed and ablate the protein are ultra-short pulse laser beams,
the ultra-short pulse laser beams are irradiated on a chip having the protein fixed thereon, protein is atomized into constituting element and ionized simultaneously by ablating the protein fixed on the chip by the ultra-short pulse laser beams, and
the ionized constituting elements are analyzed.

2. The method of analyzing protein using laser ablation according to Claim 1, wherein
said chip having the protein fixed thereon is a chip having particular protein fixed thereon in which the particular protein reacted with and bonded a substance having specific bond to the particular protein fixed on the chip.

3. The method of analyzing protein using laser ablation according to Claim 2, wherein
the substance having specific bond to said particular protein is a molecule having specific bonding characteristic with protein.

4. The method of analyzing protein using laser ablation according to Claim 3, wherein
the molecule having specific bonding characteristic with said protein is nucleic acid having specific bond with protein.

5. The method of analyzing protein using laser ablation according to Claim 2, wherein
the substance having specific bond to said particular protein is protein that exerts a specific bonding action among protein.

6. The method of analyzing protein using laser ablation according to Claim 5, wherein
the protein that exerts the specific bonding action among said protein is an antibody.

7. The method of analyzing protein using laser ablation according to Claim 6, wherein
the chip having said protein fixed thereon is formed by pouring solution containing protein that reacts with said antibody on a chip having said antibody fixed thereon, allowing protein that reacts with said antibody to react with said antibody, and allowing the protein that reacts with said antibody to bond said antibody.

8. The method of analyzing protein using laser ablation according to any one of Claims 1 to 7, wherein
an element label is attached to the protein fixed on said chip.

9. The method of analyzing protein using laser ablation according to Claim 8, wherein
said element label is a stable isotopic element label.

10. The method of analyzing protein using laser ablation according to any one of Claims 8 to 9, wherein
said element label is labeled by using a puromycin derivative.

11. The method of analyzing protein using laser ablation according to any one of Claims 8 to 9, wherein
said element label is labeled by a sandwich method.

12. The method of analyzing protein using laser ablation according to any one of Claims 8 to 9, wherein
said element label is directly labeled to protein in a sample.

13. The method of analyzing protein using laser ablation according to any one of Claims 8 to 12, wherein
said chip is a multi-channeled chip.

14. The method of analyzing protein using laser ablation according to any one of Claims 1 to 10 and 13, wherein
a sample containing protein to be analyzed and labeled protein solution, in which a label is attached to the protein to be analyzed, are mixed and poured on said chip, competitive assay is performed in which a substance having specific bond to particular protein fixed on said chip, said protein to be analyzed, and said labeled protein are bonded competitively, and the particular protein is fixed on said chip.

15. The method of analyzing protein using laser ablation according to any one of Claims 1 to 14, wherein
ultra-short pulse laser beams that are irradiated on the protein to be analyzed and ablate the protein has a pulse time width of 10 pico seconds or less and a peak value output of 10 mega watts or more.

16. The method of analyzing protein using laser ablation according to Claim 15, wherein
ultra-short pulse laser beams that are irradiated on the protein to be analyzed and ablate the protein has a pulse time width of 1 femto second or more and a peak value output of 1 giga watt or more and 10 giga watts or less.

17. The method of analyzing protein using laser ablation according to any one of Claims 1 to 16, wherein
by moving at least one of the ultra-short pulse laser beams that ablate protein and protein to be analyzed, the ultra-short pulse laser beams that ablate protein ablate the protein to be analyzed without omission and duplication to perform analysis.

18. The method of analyzing protein using laser ablation according to any one of Claims 1 to 17, wherein
the analysis of said ionized constituting elements is mass spectrometry by a time-of-flight method.

19. The method of analyzing protein using laser ablation according to any one of Claims 1 to 6, wherein
substances to be fixed on said chip are fixed as a mixture, solution attached with a different label for a substance that needs to be measured is allowed to react with the mixture, and plural types of substances are detected from the mixture.

20. The method of analyzing protein using laser ablation according to any one of Claims 1 to 6, wherein
a sample is fixed on said chip, antibody to a measuring subject, which has been labeled in plural types, is poured to measure a plurality of substances.
